# EUROPÄISCHE PATENTSCHRIFT

(11) **EP 0 722 946 B1**
(45) Veröffentlichungstag und Bekanntmachung des Hinweises auf die Patenterteilung: **19.08.1998**
(21) Anmeldenummer: 95120711.7
(22) Anmeldetag: 29.12.1995
(51) Int. Cl.: C07D 501/04, C07D 211/94, C07D 491/113

(54) **Verfahren zur Herstellung von 3-Formyl-Cephem-Derivaten**
Process for the separation of 3-formyl-cephem derivatives
Procédé pour la préparation de dérivés de 3-formyl-céphem

(30) Priorität: 12.01.1995 CH 93/95
(43) Veröffentlichungstag der Anmeldung: 24.07.1996
(73) Patentinhaber: F. HOFFMANN-LA ROCHE AG, 4070 Basel (CH)
(72) Erfinder: Lohri, Bruno, CH-4303 Kaiseraugst (CH); Vogt, Peter, CH-4142 Münchenstein (CH)
(74) Vertreter: Kjellsaa-Berger, Hanny, Dr.

(56) Entgegenhaltungen:
- EP-A- 0 445 821
- GB-A- 1 356 437
- US-A- 3 984 401
- J. ORG. CHEM. (JOCEAH,00223263);87; VOL.52 (12); PP.2559-62, UNIV. MILANO;DIP. CHIM. ORG. IND.; MILAN; ITALY (IT), LUCIO ANELLI P ET AL 'Fast and selective oxidation of primary alcohols to aldehydes or to carboxylic acids and of secondary alcohols to ketones mediated by oxoammonium salts under two-phase conditions'
- J. ORG. CHEM., Bd. 55, Nr. 2, 1990 Seiten 462-466, INOKUCHI ET AL. 'A Selective and efficient method for alcohol oxidations mediated by ...'
- J. ORG. CHEM., Bd. 56, Nr. 7, 1991 Seiten 2416-2421, INOKUCHI ET AL. 'Indirect electrooxidation of alcohols by a double mediatory system with two redox ...'

## Beschreibung

Die vorliegende Erfindung betrifft ein neues Verfahren zur Herstellung von 3-Formyl-Cephem-Derivaten der allgemeinen Formel I 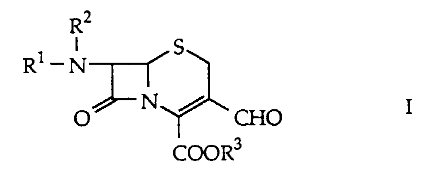 worin
- R¹: Wasserstoff oder eine Amino-Schutzgruppe;
- R²: Wasserstoff oder eine Amino-Schutzgruppe; und
- R³: eine Schutzgruppe der Carbonsäure bedeuten;
durch Oxidation des entsprechenden 3-Hydroxymethyl-Cephem Derivats.

Ein bekanntes Verfahren zur Herstellung von 3-Formyl-Cephem-Derivaten besteht darin, dass die entsprechenden 3-Hydroxymethylverbindungen mit aliphatischen Sulfoxiden in Gegenwart von Carbonsäureanhydriden oxidiert werden ( DE 2128605). Dieses Verfahren hat jedoch gewisse Nachteile, da sowohl Isomerisierungsreaktionen der Doppelbindung im Cephemring als auch Lactonbildungen auftreten können. Wird als aliphatisches Sulfoxid Dimethylsulfoxid eingesetzt, so ist der sehr unangenehme Geruch des Reaktionsprodukts Dimethylsulfid kritisch.

Der vorliegenden Erfindung liegt nun die Aufgabe zugrunde, ein Verfahren zur Herstellung von 3-Formyl-Cephem-Derivaten bereitzustellen, das die Nachteile der vorbekannten Arbeitsweise nicht aufweist.

Das erfindungsgemässe Verfahren ist dadurch gekennzeichnet, dass man ein 3-Hydroxymethyl-Cephem-Derivat der allgemeinen Formel II 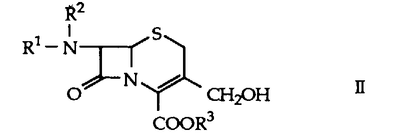 worin R¹, R² und R³ die obigen Bedeutungen haben;
mit einem anorganischen Hypohalogenit oder Halogenit in Gegenwart einer Verbindung der allgemeinen Formel III 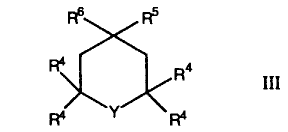 worin
- R⁴: gleiche oder verschiedene geradkettige oder verzweigte Alkylgruppen mit 1 bis 7 Kohlenstoffatomen;
- R⁵ und R⁶: entweder beide Reste Wasserstoff oder Alkoxy mit 1 bis 7 Kohlenstoffatomen bedeuten, oder der eine Rest Wasserstoff und der andere Hydroxy, Alkoxy mit 1 bis 7 Kohlenstoffatomen, Alkylcarbonyloxy mit bis 9 Kohlenstoffatomen im Alkylteil Phenylcarbonyloxy, worin der Phenylring gegebenenfalls mit C1-C7-Alkyl oder C1-C7-Alkoxyresten substituiert sein kann, oder NH-CO-C₁-C₇-Alkyl; oder R⁵ und R⁶ gemeinsam Ketal-Gruppierungen der allgemeinen Formel IVa-IVc 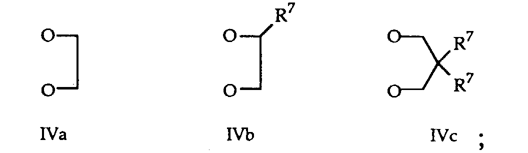
- R⁷: gleiche oder verschiedene Alkylgruppen mit 1 bis 7 Kohlenstoffatomen;
- Y: eine Gruppierung der allgemeinen Formel Va-Vc 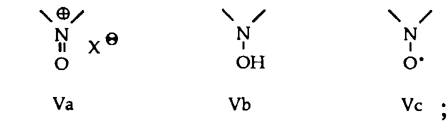 und
- X⁻: ein Anion bedeuten;
oxidiert.

Die Oxidation von Alkoholen zu Aldehyden mit Natriumhypochlorit als Oxidationsmittel und 4-Methoxy-2,2,6,6,-tetramethylpiperidin-1 oxid als Katalysator ist im Prinzip bekannt ( J. Org. Chem. Vol. 52, Nr. 12, 1987, Seite 2559-2562). Hinsichtlich der Umsetzung von ungesättigten Alkoholen wird jedoch auf die durch Nebenreaktionen bedingte unzureichende Selektivität und schlechte Ausbeute dieser Oxidationsreaktion hingewiesen (Organic Syntheses, Coll, Vol 8, 1993 p.369). Es ist somit überraschend, dass die erfindungsgemässe Oxidation von ungesättigten Alkoholen der allgemeinen Formel II mit hoher Selektivität und hohen Ausbeuten verläuft.

Der Ausdruck "Amino- Schutzgruppe" beinhaltet Schutzgruppen, die üblicherweise benutzt werden, um ein Proton oder beide Protonen der Aminogruppe zu ersetzen. Beispiele für derartige Gruppen sind beschrieben in Green T. Protective Groups in Organic Synthesis, Chapter 5, John Wiley and Sons, Inc. (1981), pp.218-287. Bekannte Beispiele für solche Schutzgruppen sind: Benzylcarbonyl, Benzyloxycarbonyl, tert.- Butyloxycarbonyl, Trimethylsilyl, Trimethylsilyethoxycarbonyl, Trichlorethoxycarbonyl, o-Nitrophenylsulfenyl, Phthaloyl und dergleichen. Bevorzugte Schutzgruppen sind tert. Butyloxycarbonyl (BOC) und Benzylcarbonyl.

Der Ausdruck "Schutzgruppe der Carbonsäure" beinhaltet Schutzgruppen, die üblicherweise benutzt werden, um ein Proton der Carboxylgruppe zu ersetzen. Beispiele für derartige Gruppen sind beschrieben in Green T. Protective Groups in Organic Synthesis, Chapter 5, John Wiley and Sons, Inc. (1981), pp.152-192. Bekannte Beispiele für solche Schutzgruppen sind: Benzhydryl, tert. Butyl, p-Nitrobenzyl, p-Methoxybenzyl, Methoxymethyl und dergleichen. Eine bevorzugte Schutzgruppe ist Benzhydryl.

Der Ausdruck "niederes Alkyl" umfasst geradkettige oder verzweigte gesättigte Kohlenwasserstoffreste mit bis zu 7 Kohlenstoffatomen, wie Methyl, Ethyl, n-Propyl, Isopropyl, n-Butyl, sec.Butyl, Isobutyl, n-Pentyl, n-Hexyl und dergleichen.

Der Ausdruck "niederes Alkoxy" bezeichnet über ein Sauerstoffatom gebundene niedere Alkylgruppen im Sinne der vorstehenden Definition, wie z.B. Methoxy, Butyloxy, Hexyloxy.

Der Ausdruck "Alkylcarbonyloxy" bezeichnet über ein Sauerstoffatom gebundene Alkylcarbonylreste. Der Ausdruck "Alkylcarbonyl" bezeichnet über eine Carbonylgruppe gebundene Alkylgruppen und umfasst sowohl niedere Alkylcarbonylgruppen, wie Acetyl oder Propionyl und dergleichen, als auch längere Alkylcarbonylgruppen, wie z.B. Caprinoyl und dergleichen.

Der Ausdruck "Arylcarbonyloxy" bezeichnet über ein Sauerstoffatom gebundene Arylcarbonylreste. Der Ausdruck "Arylcarbonyl" bezeichnet über eine Carbonylgruppe gebundene Arylgruppen. Der Ausdruck "Aryl" bezeichnet über ein Kohlenstoffatom gebundene sechsgliedrige, aromatische, gegebenenfalls substituierte cyclische Reste, wie beispielsweise einen gegebenenfalls substituierten Phenylrest, wobei als Substituenten beispielsweise niedere Alkylgruppen oder niedere Alkoxygruppen in Betracht kommen können.

Erfindungsgemäss kann ein 3-Hydroxymethyl-Cephem-Derivat der allgemeinen Formel II in einem organischen Lösungsmittel ganz oder teilweise gelöst werden.

Geeignete Lösungsmittel können im Rahmen der vorliegenden Erfindung Methylenchlorid, Essigester, Chloroform, Butylacetat, Diethylether, tert.Butylmethylether, Dichlorethan und dergleichen sowie Gemische solcher Lösungsmittel untereinander oder mit Tetrahydrofuran sein. Bevorzugt ist Dichlormethan und Essigester.

Als Oxidationsmittel dienen anorganische Hypohalogenite wie z.B. Natriumhypochlorit, Kaliumhypochlorit, Calciumhypochlorit, Natriumhypobromit und dergleichen oder Halogenite wie z.B. Natrium- oder Kaliumbromit ( J. Org. Chemistry, Vol 56, 1991 Seite 2416-2421).

Das Oxidationsmittel kann in Form von wässrigen Lösungen eingesetzt werden oder auch durch elektrochemische Reaktion in situ gebildet werden, z.B. in der Art wie in Chemistry Letters, 1994, Seite 1411-1414 beschrieben.

Für die vollständige Oxidation von 3-Hydroxymethyl-Cephem-Derivat zu 3-Formyl-Cephem-Derivat sind z.B. im Fall von Hypochlorit als Oxidationsmittel mindestens äquimolare Mengen des Hypochlorits bezogen auf die Menge von 3-Hydroxymethyl-Cephem-Derivat erforderlich, jedoch empfiehlt es sich im allgemeinen das Hypochlorit in einem Ueberschuss von bis zu 100% einzusetzen.

Die Oxidation wird in Gegenwart von Verbindungen der allgemeinen Formel III als Katalysator durchgeführt. Der Katalysatorzusatz ist für das erfindungsgemässe Oxidationsverfahren essentiell. Bevorzugt als Katalysator sind 2,2,6,6,-Tetramethylverbindungen der Formel III. Besonders bevorzugt ist 2,2,6,6-Tetramethylpiperidin-1-oxyl-Radikal (TEMPO).

Die Herstellung der Verbindungen der allgemeinen Formel III wird beispielsweise in Synthesis, 1971, S.190 ff beschrieben.

Die Oxidation von 3-Hydroxymethyl-Cephem Derivativen der Formel II wird in Gegenwart von etwa 1 bis etwa 20 mol%, vorzugsweise von etwa 3 bis etwa 7 mol% einer Verbindung der Formel III bezogen auf das Ausgangsmaterial durchgeführt

Das Oxidationssystem beinhaltet die Verwendung eines Puffers, wie z.B. Natriumhydrogencarbonat, Natriumacetat, Dinatriumhydrogen-phosphat und dergleichen.

Die Oxidationsreaktion wird durch Bromidionen begünstigt, welche man zweckmässigerweise in Form von Natrium - oder Kaliumbromid einsetzt in Mengen von 1-20 Mol% bezüglich 3-Hydroxymethyl-Cephem-Derivat, sofern es sich nicht um ein bromhaltiges Oxidationsmittel handelt, wo sich der Zusatz von Bromid erübrigt.

Die Oxidation kann zweckmässigerweise in einem Temperaturbereich von -15° C - 50° C , vorzugsweise bei 0° C - 5°C erfolgen. Zweckmässigerweise kann man unter Atmosphärendruck arbeiten.

Zweckmässigerweise werden alle Reaktanden mit Ausnahme des Oxidationsmittels vorgelegt, und das Oxidationsmittel wird zudosiert.

Die Aufarbeitung des Rohproduktes an 3-Formyl-Cephem-Derivaten kann durch Extraktion mit Lösungsmitteln, wie Methylenchlorid, Essigester, tert.Butylmethylether und dergleichen, erfolgen. Weitere Reinigung kann durch Rekristallisation oder Umfällung erfolgen.

Mittels des erfindungsgemässen Verfahrens können Umsetzungen von 3-Hydroxymethyl-Cephem-Derivaten zu 3-Formyl-Cephem-Derivaten mit hohen, häufig nahezu quantitativen Ausbeuten erzielt werden.

Diese 3-Formyl-Cephem-Derivate bilden wertvolle Zwischenprodukte bei der Synthese von Cephalosporinderivaten. Beispielsweise können Cephalosporinderivate der allgemeinen Formel VI hergestellt werden, 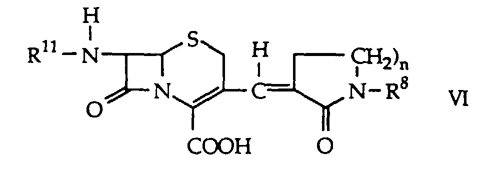 worin
- R¹¹: eine in der Cephalosporinchemie gebräuchliche, von einer Carbonsäure abgeleitete Acylgruppe bedeutet,
- R⁸: Wasserstoff, Hydroxy, niederes Alkyl-Qₘ, Cycloalkyl, niederes Alkoxy, niederes Alkenyl, Cycloalkenyl, niederes Alkinyl, Aralkyl-Qₘ, Aryl-Qₘ, Aryloxy, Aralkoxy oder einen heterocyclischen Ring bedeutet;
- Qm: -CO- oder -SO₂ bedeutet;
- m: 0 oder 1 bedeutet;
- n: 0, 1 oder 2 bedeutet,
wie in EP-A-0 620 225 beschrieben.

Die folgenden Beispiele erläutern die Erfindung näher, sollen aber keinerlei Einschränkung darstellen.

### Beispiel 1:

Herstellung von (6R,7R)-7-[(1, 1-Dimethylethoxy)carbonylamino]-3-formyl-8-oxo-5-thia-1-aza-bicyclo[4.2.0.]oct-2-en-2-carbonsäure-benzhydrylester unter Verwendung von Dichlormethan als Lösungsmittel.
[R¹ = CH₃-C(CH₃)₂-O-CO-, R² = H, R³ = (C₆H₅)₂-CH-]
3.0 g (6 mMol) (6R,7R)-7-[(1,1-Dimethylethoxy)carbonylamino]-3-hydroxymethyl-8-oxo-5-thia-1-aza-bicyclo[4.2.0.]oct-2-en-2-carbonsäure-benzhydrylester wurden unter Erwärmen auf 35°C in 24 ml Dichlormethan gelöst. Nach Abkühlen der Lösung auf 22°C wurde eine Lösung von 90 mg (0.76 mMol) Kaliumbromid und 240 mg (2.86 mMol) Natriumbicarbonat in 24 ml Wasser hinzugefügt. Unter kontinuierlichem Rühren (ca. 600 Upm) wurde die Lösung auf 0°C abgekühlt und dann mit 60 mg (0.38 mMol) 2,2,6,6-Tetramethylpiperidin-1-oxyl-Radikal( TEMPO) versetzt. Bei 0-2°C wurden innerhalb von 1 Std. 3.6 ml (7.2 mMol) 15-proz. (g/v) wässrige Natriumhypochlorit-Lösung zudosiert. Das Rühren des Reaktionsgemisches wurde noch 30 Min. fortgesetzt, dann war die Reaktion gemäss Dünnschichtanalyse beendet. Die Aufarbeitung des Reaktionsgemisches erfolgte in der üblichen Art durch Extraktion mit Dichlormethan/Wasser und Waschen der organischen Phasen mit verdünnter Kochsalzlösung. Trocknen und Eindampfen der organischen Phasen führte zu 3.2 g Rohprodukt mit einem Gehalt von 94.4 % (HPLC Flächenproz.) an (6R,7R)-7-[(1,1-Dimethylethoxy)carbonylamino]-3-formyl-8-oxo-5-thia-1-aza-bicyclo[4.2.0.]oct-2-en-2-carbonsäure-benzhydrylester. Umkristallisation des Rohprodukts ergab 2.49 g (83 % d.Th.) weisse Kristalle, Smp. 172-173°C, mit einem Gehalt an (6R,7R)-7-[(1,1-Dimethylethoxy)carbonylamino]-3-formyl-8-oxo-5-thia-1-aza-bicyclo[4.2.0.]oct-2-en-2-carbonsäure-benzhydrylester von 99.0 % (HPLC Flächenproz.).

### Beispiel 2:

Herstellung von (6R,7R)-7-[(1,1-Dimethylethoxy)carbonylamino]-3-formyl-8-oxo-5-thia-1-aza-bicyclo[4.2.0.]oct-2-en-2-carbonsäure-benzhydrylester unter Verwendung von Essigester als Lösungsmittel.
[R¹ = CH₃-C(CH₃)₂-O-CO-, R² = H, R³ = (C₆H₅)₂-CH-] 9.0 g (18 mMol) (6R,7R)-7-[(1,1-Dimethylethoxy)carbonylamino]-3-hydroxymethyl-8-oxo-5-thia-1-aza-bicyclo[4.2.0.]oct-2-en-2-carbonsäure-benzhydrylester wurden in 74 ml Essigester teilweise gelöst. Eine Lösung von 270 mg (2.3 mMol) Kaliumbromid und 720 mg (8.6 mMol) Natriumbicarbonat in 66 ml Wasser wurde hinzugefügt. Das Gemisch wurde unter kontinuierlichem Rühren auf 0°C abgekühlt und dann mit einer Lösung von 180 mg (1.15 mMol) 2,2,6,6-Tetramethylpiperidin-1-oxyl- Radikal ( TEMPO) in 1 ml Essigester versetzt. Bei 0-2°C wurden innerhalb von 1 Std. 9.15 ml (21.8 mMol) 17.7-proz. (g/v) wässrige Natriumhypochlorit-Lösung zudosiert. Das Rühren des Reaktionsgemisches wurde noch 15 Min. fortgesetzt, dann war die Reaktion gemäss Dünnschichtanalyse beendet. Die Aufarbeitung des Reaktionsgemisches erfolgte in der üblichen Art durch Extraktion mit Essigester/Wasser und Waschen der organischen Phasen mit verd. Kochsalzlösung. Trocknen und Eindampfen der organischen Phasen und Umkristallisation des Rohprodukts ergab 8.1 g (91 % d.Th.) weisse Kristalle mit einem Gehalt von 96.4 % (HPLC Flächenproz.) an (6R,7R)-7-[(1,1-Dimethylethoxy)carbonylamino]-3-formyl-8-oxo-5-thia-1-azabicyclo[4.2.0.]oct-2-en-2-carbonsäure-benzhydrylester.

### Beispiel 3:

Herstellung von (6R,7R)-7-Benzylcarbonylamino-3-formyl-8-oxo-5-thia-1-azabicyclo[4.2.0.]oct-2-en-2-carbonsäure-benzhydrylester
[R¹ = (C₆H₅)-CH₂-CO-, R² = H, R³ = (C₆H₅)₂-CH-]
9.3 g (18 mMol) (6R,7R)-7-Benzylcarbonylamino-3-hydroxymethyl-8-oxo-5-thia-1-aza-bicyclo[4.2.0.]oct-2-en-2-carbonsäure-benzhydrylester wurden in einem Gemisch von 24 ml Dichlormethan und 12 ml THF teilweise gelöst. Eine Lösung von 270 mg (2.3 mMol) Kaliumbromid und 720 mg (8.6 mMol) Natriumbicarbonat in 66 ml Wasser wurde hinzugefügt. Das Gemisch wurde unter kontinuierlichem Rühren auf 0°C abgekühlt und dann mit 105 mg (0.67 mMol) 2,2,6,6-Tetramethylpiperidin-1-oxyl-Radikal ( TEMPO ) versetzt. Bei 0-2°C wurden innerhalb von 1 Std. 9 ml (21.6 mMol) 17.9-proz. (g/v) wässrige Natriumhypochlorit-Lösung zudosiert. Das Rühren des Reaktionsgemisches wurde noch 15 Min. fortgesetzt, dann war die Reaktion gemäss Dünnschichtanalyse beendet. Die Aufarbeitung des Reaktionsgemisches erfolgte in der üblichen Art durch Extraktion mit Dichlormethan/Wasser. Trocknen und Eindampfen der organischen Phasen und Umfällen des Rohprodukts ergab 8.9 g (96 % d.Th.) weisses Pulver, Smp. 130-132°C, mit einem Gehalt von 98.7 % (HPLC Flächenproz.) an (6R,7R)-7-Benzylcarbonylamino-3-formyl-8-oxo-5-thia-1-aza-bicyclo[4.2.0.] oct-2-en-2-carbonsäure-benzhydrylester.

### Beispiele 4-12:

Oxidation von (6R,7R)-7-[(1,1-Dimethylethoxy)carbonylamino]-3-hydroxymethyl-8-oxo-5-thia-1-aza-bicyclo[4.2.0.]oct-2-en-2-carbonsäurebenzhydrylester in Gegenwart von weiteren Katalysatoren der allgemeinen Formel III

(6R,7R)-7-[(1,1-Dimethylethoxy)carbonylamino]-3-hydroxymethyl-8-oxo-5-thia-1-aza-bicyclo[4.2.0.]oct-2-en-2-carbonsäure-benzhydrylester (Ansatzgrössen von 0.5 g bis 3.0 g) wurde in der Art und Weise wie in Beispiel 1 beschrieben mit 1.2 Moläquivalenten Natriumhypochlorit-Lösung zu (6R,7R)-7-[(1,1-Dimethylethoxy)carbonylamino]-3-formyl-8-oxo-5-thia-1-aza-bicyclo[4.2.0.]oct-2-en-2-carbonsäure-benzhydrylester, dem entsprechenden 3-Formylcephemderivat, umgesetzt. Im Unterschied zu Beispiel 1 wurden anstatt TEMPO weitere Katalysatoren der allgemeinen Formel III eingesetzt, und zwar in einer Menge von 6.4 Molproz. bezgl. Ausgangsmaterial, wenn nichts anderes erwähnt ist.

Die eingesetzten Katalysatoren und erzielten Ausbeuten an 3-Formylcephemderivat sind in den Tabellen 1 und 2 zusammengefasst.

**Tabelle 1**

| | Katalysator R⁴ = CH₃, R⁵ = H Y = Formel Vc | % 3-Formyl-cephemderivat (Flächenproz. HPLC) |
|---|---|---|
| Beispiel 4 | R⁶ = OCH₃ | 90.4 |
| Beispiel 5 | R⁶ = O(CH₂)₅-CH₃ | 87.7 |
| Beispiel 6 | R⁶ = O(CH₂)₃-CH₃ | 87.1 |
| Beispiel 7 | R⁶ = NH-COCH₃ ¹⁾ | 82.8 |
| Beispiel 8 | R⁶ = OCO(CH₂)₈-CH₃ | 81.0 |
| Beispiel 9 | R⁶ = OCOCH(CH₃)₂ | 78.9 |

| | | |
|---|---|---|
| ¹⁾ 4.7 Molproz. | | |

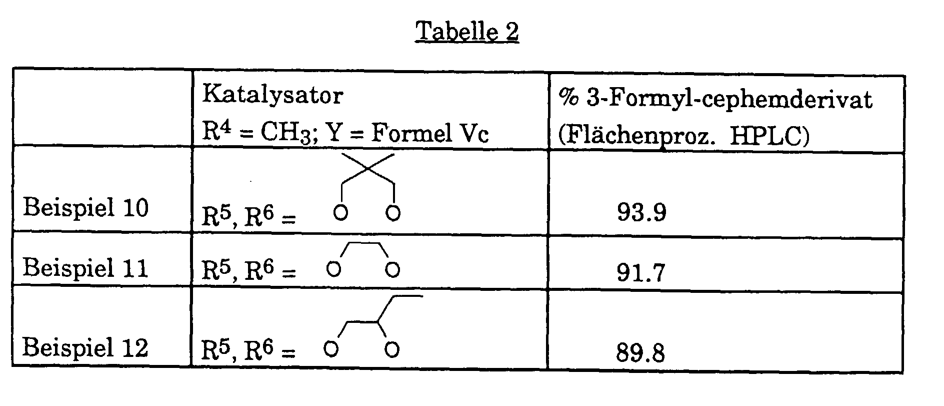

## Patentansprüche

1. Verfahren zur Herstellung von 3-Formyl-Cephem-Derivaten der allgemeinen Formel I 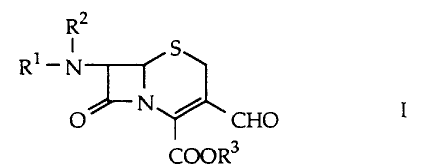 worin
R¹ Wasserstoff oder eine Amino-Schutzgruppe;
R² Wasserstoff oder eine Amino-Schutzgruppe; und
R³ eine Schutzgruppe der Carbonsäure bedeuten;
dadurch gekennzeichnet, dass man ein 3-Hydroxymethyl-Cephem-Derivat der allgemeinen Formel II 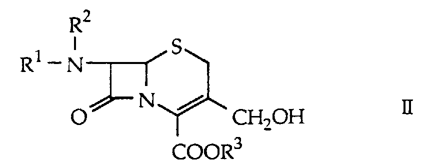 worin R¹, R² und R³ die obigen Bedeutungen haben;
mit einem anorganischen Hypohalogenit oder Halogenit in Gegenwart von Verbindungen der allgemeinen Formel III 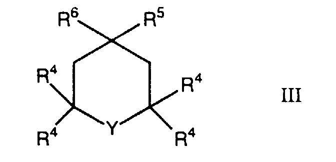 worin
R⁴ gleiche oder verschiedene geradkettige oder verzweigte Alkylgruppen mit 1 bis 7 Kohlenstoffatomen;
R⁵ und R⁶ entweder beide Reste Wasserstoff oder Alkoxy mit 1 bis 7 Kohlenstoffatomen bedeuten, oder der eine Rest Wasserstoff und der andere Hydroxy, Alkoxy mit 1 bis 7 Kohlenstoffatomen, Alkylcarbonyloxy mit bis 9 Kohlenstoffatomen im Alkylteil, Phenylcarbonyloxy, worin der Phenylring gegebenenfalls mit C1-C7-Alkyl oder C1-C7-Alkoxyresten substitutiert sein kann, oder NH-CO-C₁-C₇-Alkyl; oder R⁵ und R⁶ gemeinsam Ketal-Gruppierungen der allgemeinen Formel IVa-IVc 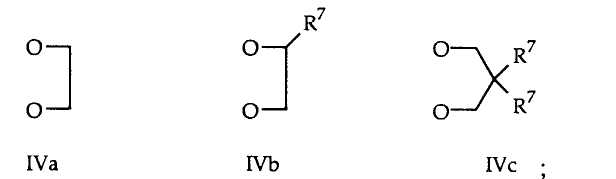
R⁷ gleiche oder verschiedene Alkylgruppen mit 1 bis 7 Kohlenstoffatomen; und
Y eine Gruppierung der allgemeinen Formel Va-Vc 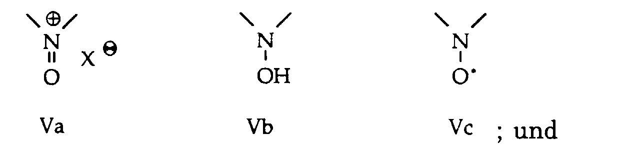
X⁻ ein Anion bedeuten;
oxidiert

2. Verfahren gemäss Anspruch 1, dadurch gekennzeichnet, dass die Verbindung der allgemeinen Formel III 2,2,6,6,-Tetramethylpiperidin-1-oxid oder 4-Methoxy-2,2,6,6,-tetramethylpiperidin-1-oxid ist.

3. Verfahren gemäss Anspruch 1 oder 2, dadurch gekennzeichnet, dass die Verbindung der allgemeinen Formel III in einer Menge von 1-10 Mol%, insbesondere 3-7 Mol% eingesetzt wird.

4. Verfahren gemäss einem der Ansprüche 1-3, dadurch gekennzeichnet, dass als Oxidationsmittel Natriumhypochlorit in einer Menge von 1-2 Molequivalent eingesetzt wird.

5. Verfahren gemäss einem der Ansprüche 1-4, dadurch gekennzeichnet, dass die Oxidation in Gegenwart von KBr und NaHCO₃ durchführt wird.

6. Verfahren gemäss einem der Ansprüche 1-5, dadurch gekennzeichnet, dass die Oxidation in Gegenwart von Dichlormethan durchführt wird.

7. Verfahren gemäss einem der Ansprüche 1-5, dadurch gekennzeichnet, dass die Oxidation in Gegenwart von Essigester durchführt wird.

8. Verfahren gemäss einem der Ansprüche 1-7, dadurch gekennzeichnet, dass die Reaktion bei einer Temperatur von -15° C - +50° C , vorzugsweise bei 0° C - +5° C durchgeführt wird.

## Claims

1. A process for the manufacture of 3-formyl-cephem derivatives of general formula I 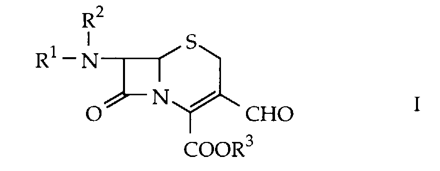 wherein
R¹ signifies hydrogen or an amino protecting group;
R² signifies hydrogen or an amino protecting group; and
R³ signifies a carboxylic acid protecting group;
characterized by oxidizing a 3-hydroxymethyl-cephem derivative of general formula II 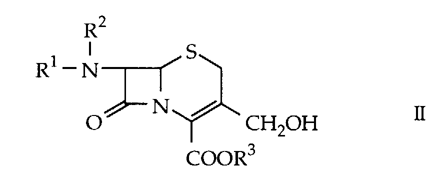 wherein R¹, R² and R³ have the above significances;
with an inorganic hypohalite or halite in the presence of compounds of general formula III 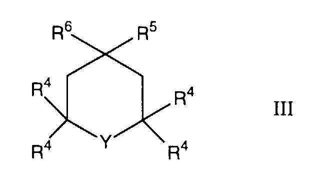 wherein
R⁴ signify the same or different straight-chain or branched alkyl groups with 1 to 7 carbon atoms;
R⁵ and R⁶ either both signify hydrogen or alkoxy with 1 to 7 carbon atoms or one signifies hydrogen and the other signifies hydroxy, alkoxy with 1 to 7 carbon atoms, alkylcarbonyloxy with up to 9 carbon atoms in the alkyl part, phenylcarbonyloxy, in which the phenyl ring can be optionally substituted with C1-C7 alkyl or C1-C7 alkoxy residues, or NH-CO-C₁-C₇-alkyl; or R⁵ and R⁶ together signify ketal groupings of general formula IVa-IVc 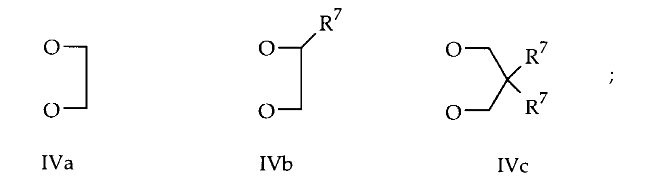
R⁷ signify the same or different alkyl groups with 1 to 7 carbon atoms;
Y signifies a grouping of general formula Va-Vc 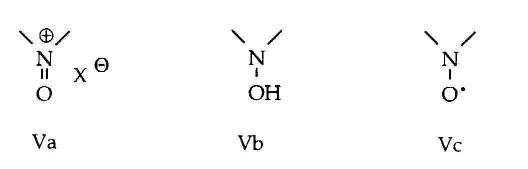
and X⁻ signifies an anion.

2. A process according to claim 1, characterized in that the compound of general formula III is 2,2,6,6-tetramethylpiperidine 1-oxide or 4-methoxy-2,2,6,6-tetramethylpiperidine 1-oxide

3. A process according to claim 1 or 2, characterized in that the compound of general formula III is used in an amount of 1-10 mol%, especially 3-7 mol%.

4. A process according to any one of claims 1-3, characterized in that sodium hypochlorite is used as the oxidizing agent in an amount of 1-2 mol equivalents.

5. A process according to any one of claims 1-4, characterized in that the oxidation is carried out in the presence of KBr and NaHCO₃.

6. A process according to any one of claims 1-5, characterized in that the oxidation is carried out in the presence of dichloromethane.

7. A process according to any one of claims 1-5, characterized in that the oxidation is carried out in the presence of ethyl acetate.

8. A process according to any one of claims 1-7, characterized in that the oxidation is carried out at a temperature of -15°C - +5°C, preferably at 0°C - +5°C.

## Revendications

1. Procédé de préparation de dérivés du 3-formylcéphème répondant à la formule générale I: 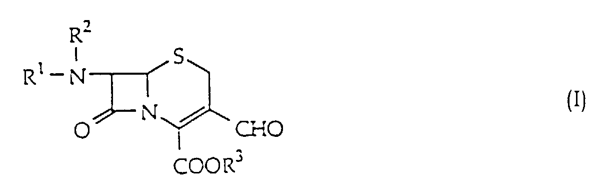 dans laquelle
R¹ représente l'hydrogène ou un groupe protecteur du groupe amino ;
R² représente l'hydrogène ou un groupe protecteur du groupe amino ; et
R³ représente un groupe protecteur de l'acide carboxylique ;
caractérisé en ce que l'on oxyde un dérivé du 3-hyroxyméthylcéphème répondant à la formule générale II : 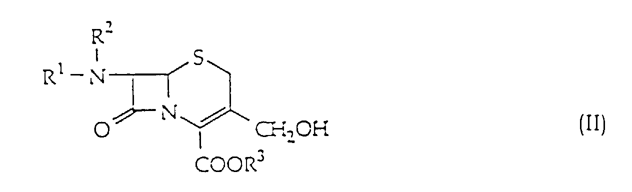 dans laquelle R¹, R² et R³ ont les significations indiquées ci-dessus,
par un hypohalogénite ou un halogénite minéral en présence de composés de formule générale III : 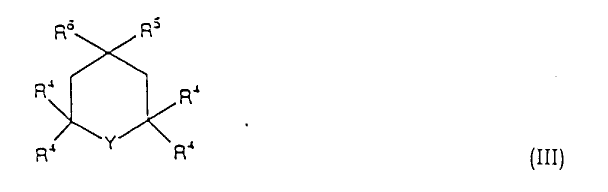 dans laquelle
les symboles R⁴, ayant des significations identiques ou différentes, représentent chacun un groupe alkyle à chaîne droite ou ramifiée en C₁-C₇ ;
R⁵ et R⁶ représentent tous deux l'hydrogène ou des groupes alcoxy en C₁-C₇, ou bien l'un de ces deux symboles représente l'hydrogène et l'autre un groupe hydroxy, alcoxy en C₁-C₇, un groupe alkylcarbonyloxy contenant jusqu'à 9 atomes de carbone dans la partie alkyle, un groupe phénylcarbonyloxy dans lequel le cycle phényle porte éventuellement des substituants alkyle en C₁-C₇ ou alcoxy en C₁-C₇, ou bien un groupe NH-CO-alkyle en C₁-C₇ ; ou bien R⁵ et R⁶ forment ensemble un groupement acétal de formule générale IVa à IVc 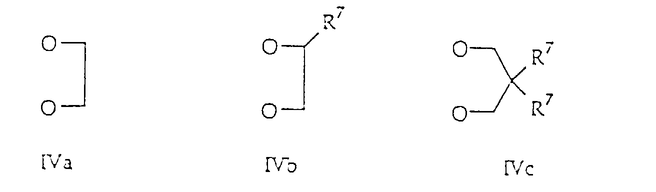 dans lesquelles
les symboles R⁷, ayant des significations identiques ou différentes, représentent chacun un groupe alkyle en C₁-C₇ ; et
Y représente un groupement de formule générale Va à Vc 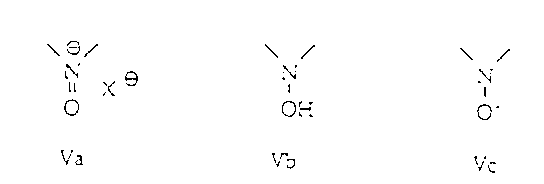 et X⁻ représente un anion.

2. Procédé selon la revendication 1, caractérisé en ce que le composé de formule générale III est le 1-oxyde de la 2,2,6,6-tétraméthylpipéridine ou le 1-oxyde de la 4-méthoxy-2,2,6,6-tétraméthylpipéridine.

3. Procédé selon la revendication 1 ou 2, caractérisé en ce que le composé de formule générale III est mis en oeuvre en quantité de 1 à 10 mol %, plus spécialement de 3 à 7 mol %.

4. Procédé selon l'une des revendications 1 à 3, caractérisé en ce que l'on utilise en tant qu'agent oxydant l'hypochlorite de sodium de 1 à 2 équivalents molaires.

5. Procédé selon l'une des revendications 1 à 4, caractérisé en ce que l'oxydation est réalisée en présence de KBr et de NaHCO₃.

6. Procédé selon l'une des revendications 1 à 5, caractérisé en ce que l'oxydation est réalisée en présence de dichlorométhane.

7. Procédé selon l'une des revendications 1 à 5, caractérisé en ce que l'oxydation est réalisée en présence d'acétate d'éthyle.

8. Procédé selon l'une des revendications 1 à 7, caractérisé en ce que la réaction est exécutée à une température allant de -15 à +50°C, de préférence de 0 à +5°C.
